# EUROPÄISCHE PATENTSCHRIFT

(11) **EP 2 309 943 B1**
(45) Veröffentlichungstag und Bekanntmachung des Hinweises auf die Patenterteilung: **13.01.2016**
(21) Anmeldenummer: 09742056.6
(22) Anmeldetag: 05.05.2009
(51) Int. Cl.: A61B 19/00, A61B 19/02

(54) **MARKER FÜR EIN NAVIGATIONSSYSTEM**
MARKER FOR A NAVIGATION SYSTEM
MARQUEUR POUR SYSTÈME DE NAVIGATION

(30) Priorität: 06.05.2008 DE 102008022254
(43) Veröffentlichungstag der Anmeldung: 20.04.2011
(73) Patentinhaber: Atesos engineering AG, 5000 Aarau (CH)
(72) Erfinder: Hauri, Bernhard, 5053 Staffelbach (CH); Hauri, Thomas, 5053 Staffelbach (CH)
(74) Vertreter: Popp, Eugen
(86) Internationale Anmeldenummer: PCT/EP2009/055413
(87) Internationale Veröffentlichungsnummer: WO 2009/135838

(56) Entgegenhaltungen:
- EP-A- 1 639 958
- EP-A- 1 658 819
- WO-A-03/020146
- US-A1- 2007 225 599

## Beschreibung

Die vorliegende Erfindung betrifft einen Marker für ein Navigationssystem zur Bestimmung der räumlichen Position z. B. eines chirurgischen Instruments, mit einer sphärischen, retroreflektierenden Oberfläche, insbesondere in Form einer retroreflektierenden Kugel, gemäß dem Oberbegriff des Anspruches 1.

Marker werden verwendet, um z.B. bei bildunterstützten chirurgischen Verfahren (IGS = Image Guided Surgery) die Position von mit z.B. mehreren Markern in Form eines Referenzstems verbundenen chirurgischen Instrumenten oder Körpern durch eine optische Erfassung des von den Markern reflektierten Lichtes zu ermitteln und basierend auf den ermittelten Positionsinformationen einen chirurgischen Eingriff durchzuführen.

Chirurgische Navigationssysteme dienen also der Vermessung von geometrischen Größen (meist in sterilem Umfeld) bei chirurgischen Eingriffen an Mensch und Tier. Eine weit verbreitete Meßtechnik ist - wie bereits erwähnt - die Positionsmessung von optischen Markern mit einem lichtoptischen Kamerasystem. Diese optischen Marker sind entweder selbstleuchtend (= aktive Marker), oder reflektieren Licht, welches von einem blitzleuchtenden Kamerasystem erzeugt wird (= passive Marker). Wegen der besonderen Bedingungen, insbesondere im sterilen Umfeld eines chirurgischen Eingriffes, haben passive Elemente Vorteile, da diese keine eigene Energieversorgung benötigen. Energieversorgung und leuchtende Elemente bedingen im Prozeß einer sterilen Wiederaufbereitung (Waschen in Waschmaschine oder Sterilisieren bei 134°C in Dampf) sehr hohe Anforderungen an die Beständigkeit und Konstruktion derartiger Bauteile.

Bei den passiven Markern wird das entlang der optischen Achse des Meßobjektives einfallende Licht, welches meist durch ringförmige Blitzleuchten, welche um das Kameraobjektiv herum angeordnet sind, erzeugt wird, in umgekehrter Richtung reflektiert (= Retroreflexion). Da die Positionsvermessung eines chirurgischen Instrumentes, oder einer zu vermessenden Körpergeometrie unabhängig vom Betrachtungswinkel sein soll, stellt die Verwendung kugelförmiger Marker die meßtechnisch einfachste und sicherste Lösung dar. Unabhängig von der Betrachtungsrichtung projiziert sich eine Kugel immer in einen Kreis, während flache Geometrien, wie scheibenförmige Marker, verzerrte Abbildungen bzw. Konturen, z.B. ellipsenähnliche Konturen ergeben, welche nur mit größerem Aufwand auswertbar sind. Anwendungsbedingt liegt der Durchmesser der bevorzugt verwendeten Markerkugeln zwischen 5 mm und 20 mm.

Die Retroreflexion wird durch eine speziell gestaltete Oberfläche erreicht. Bekannt sind Beschichtungen mit feinsten Prismen, oder Beschichtungen mit feinsten Glaskugeln, welche in die Oberfläche eingebettet sind. Typische Abmessungen dieser feinsten Glaskügelchen liegen zwischen 20 µ und 200 µ. Bei geeignetem Brechungsindex gegenüber der umgebenden Luft und bei geeigneten Reflexionseigenschaften der Einbettfläche, welche im Falle von Glaskügelchen einen miniaturisierten Hohlspiegel darstellt, wird das einfallende Licht retroreflektiert. Derartige Oberflächen finden in der Sicherheitstechnik breite Anwendung und werden häufig in Folienform hergestellt.

Aufgrund der Feinheit der Glaskügelchen ist eine retroreflektierende Oberfläche mikrorauh. Wird eine derartige retroreflektierende Oberfläche mit einer Substanz belegt, welche nicht transparent ist, z.B. Schmutzpartikel oder Blut, erscheint dieser Bereich in der Abbildung durch die Kamera als dunkler Fleck. Wegen der Mikrorauhigkeit ist die Entfernung derartiger Verschmutzungen aufwendig und sehr häufig auch unvollständig, da Schmutz in den Spalten zwischen den Glaskügelchen zurückbleibt. Wird eine derartige Oberfläche mit einem transparenten Medium benetzt, z.B. Wasser, verliert diese wegen des gestörten Strahlengangs "Luft-Glaskügelchen" ihre retroreflektierende Eigenschaft. Im Bild der Meßkamera erscheint eine mit Wasser benetzte retroreflektierende Oberfläche dunkel und scheidet für eine Messung aus.

Bezüglich der Gestaltung retroreflektierender Markerkugeln wird ergänzend auf die FR 2 706 045 A1 verwiesen. Soweit es um die Anwendung derartiger Markerkugeln in der Chirurgie geht, wird auf die WO 2007/090288 A1 hingewiesen.

Um die vorgenannten Nachteile durch Verschmutzung oder Benetzung retroreflektierender Oberflächen zu vermeiden, hat man diese mit einer Folie abgedeckt derart, daß diese einen geringen Abstand zur retroreflektierenden Oberfläche einhält. Schmutz od. dgl. läßt sich von der glatten Folienoberfläche leicht abwischen. Die Benetzung mit Wasser stört den Strahlengang nur unwesentlich. Der strahlenoptische Übergang "Luft-Glaskügelchen" wird durch den kleinen Luftspalt zwischen Schutzfolie und retroreflektierender Oberfläche gewährleistet. Als Abstandshalter zwischen Schutzfolie und retroreflektierender Oberfläche wird ein Wabenmuster verwendet. Solche Konstruktionen lassen sich bei ebenen Flächen noch relativ gut herstellen. Für sphärische, insbesondere kugelförmige Oberflächen sind die bekannten Schutzfolien ungeeignet. Es kommt zu nicht definierten Verzerrungen. Demzufolge ist die Genauigkeit der Positionsbestimmung begrenzt, insbesondere wenn Marker-Erkennungs-Algorithmen verwendet werden, welche auf der Detektion des Massenmittelpunktes basieren (siehe dazu auch EP 1 774 922 A1).

Aus diesem Grunde werden in der Praxis nach wie vor entweder ungeschützte Markerkugeln oder foliengeschützte, ebenflächige Marker verwendet. Wie erwähnt, haben flache, schmutzgeschützte Markerelemente den Vorteil, daß sie leicht gereinigt werden können, aber den Nachteil, daß die Abbildung, die durch eine Meßkamera festgehalten wird, verzerrt ist. Ungeschützte Markerkugeln haben den Vorteil einer meßtechnisch idealen Abbildung, aber den Nachteil leichter Verschmutzung und Störung der Retroreflexion durch Benetzung mit Wasser od. dgl. transparenten Flüssigkeit.

Aus der EP 1 658 819 A1 ist eine formstabile, transparente Markerumhüllung bekannt, die zum Schutz der Marker vor Verschmutzung dient. Die Umhüllung kann Verstärkungen, Falze oder Falten aufweisen, die so angeordnet sind, daß sie eine Detektion der Marker nicht stören. Auch aus der US 2007/225599 A1 sind formstabile, lichtdurchlässige Marker-Schutzkappen bekannt, die jeweils einen zylindrischen oder kegelstumpfförmigen Abschnitt aufweisen.

Die WO 03/020146 offenbart einem Marker unit dem Merkmalen des Oberbegriffs von Anspruch 1.

Besondere Maßnahmen für verzerrungsfreie Abbildungen, insbesondere kreisrunde Abbildungen der retroreflektierenden Oberfläche eines Markers auch im zylindrischen Abschnitt der Schutzhülle, insbesondere unter einem hohen Betrachtungswinkel von bis zu 70° sind beim Stand der Technik nicht vorgesehen.

Dementsprechend liegt der vorliegenden Erfindung die Aufgabe zugrunde, einen Marker zur Verfügung zu stellen, der trotz Schutzhülle verzerrungsfreie Abbildungen auch unter hohen Betrachtungswinkeln erlaubt.

Diese Aufgabe wird erfindungsgemäß durch die kennzeichnenden Merkmale des Anspruches 1 gelöst, wobei vorteilhafte Weiterbildungen und konstruktive Details in den Unteransprüchen beschrieben sind.

Einer der wesentlichen Aspekte der vorliegenden Erfindung ist also die Abdeckung einer sphärischen retroreflektierenden Oberfläche, insbesondere einer retroreflektierenden Kugel durch eine lichtdurchlässige bzw. transparente Schale, und zwar derart, daß die reflektierte Abbildung hinsichtlich Kontur und Schwerpunktlage durch die Schale im wesentlichen unbeeinflußt ist. Durch die erfindungsgemäße Schale soll also die meßtechnisch ideale Abbildung eines von einer Lichtquelle emittierten und von der reflektierenden Oberfläche reflektierten Lichts in einer zugeordneten Meßkamera nicht nachteilig beeinflußt werden. Ideal ist eine kreisrunde Abbildung der retroreflektierenden Oberfläche durch die Kamera. Die erfindungsgemäß verwendete Schale soll in an sich bekannter Weise formstabil sein, so daß Abstandselemente zwischen der retroreflektierenden Oberfläche und der Schale vermieden werden können. Damit läßt sich eine ungestörte Abbildung des durch die retroreflektierende Oberfläche reflektierten Lichts in der Kamera sicherstellen. Die Abbildung wird durch die formstabile, transparente Schale hinsichtlich Kontur und Schwerpunktlage nicht verändert.

Da herkömmliche Retroreflexionsoberflächen nur im Umfeld von Luft oder gasförmigen Medien mit einer Brechzahl nahe bei "1" funktionieren, ist es erforderlich, daß die die retroreflektierende Oberfläche schützende Schale in einem geringen Abstand von der retroreflektierenden Oberfläche angeordnet ist. Vorzugsweise ist die Schale von der retroreflektierenden Oberfläche im Bereich zwischen 0,1 mm bis 0,5 mm, insbesondere im Bereich zwischen etwa 0,3 mm bis 0,4 mm beabstandet.

Die Dicke der die retroreflektierende Oberfläche schützenden Schale beträgt etwa 0,7 mm bis 1,2 mm, insbesondere etwa 1,0 mm. Sie ist vorzugsweise aus hochtransparentem Kunststoff hergestellt. Auch Glas ist als Werkstoff für die Schale denkbar, auch wenn die Herstellung aus Glas aufwendiger ist.

Ein weiterer wesentlicher Aspekt der Erfindung zeichnet sich dadurch aus, daß die die retroreflektierende Oberfläche schützende Schale einteilig ausgebildet ist. Dementsprechend umfaßt die Schale eine über die retroreflektierende Oberfläche, insbesondere Kugel gestülpte Haube mit einem sphärischen Haubenabschnitt und einem sich daran anschließenden asphärischen Haubenabschnitt. Diese Ausführungsform ist hinsichtlich der Herstellung relativ einfach. Die Schutzhaube kann als Spritzgußteil hergestellt werden. Am freien Ende des asphärischen Abschnitts ist vorzugsweise ein Flansch angeformt, über den die Befestigung an einem nicht notwendigerweise mehr transparenten Tragteller erfolgt. Dieser Tragteller ist an einen Tragstift oder Trägeradapter des Navigationssystems anschließbar, insbesondere aufsteckbar.

Um auch im Bereich des asphärischen Abschnitts verzerrungsfreie Abbildungen zu erhalten, insbesondere eine kreisrunde Abbildung der retroreflektierenden Oberfläche, weist dieser Abschnitt optische Korrekturbereiche auf, z.B. in Form einer äußeren Umfangsfläche, die sich in axialer Richtung ausgehend vom oder unterhalb des Übergangs zwischen diesem Abschnitt und sphärischem Haubenabschnitt konisch nach außen erstreckt. Dabei ist zu bedenken, daß Retroreflexionskugeln in der Praxis bis zu einem Betrachtungswinkel von etwa 70° relativ zu der durch den Trägerstift definierten Mittenachse eingesetzt werden. Durch die erwähnten Maßnahmen im asphärischen Abschnitt läßt sich auch im Bereich eines Betrachtungswinkels von nahe 70° noch eine kreisrunde Abbildung der retroreflektierenden Oberfläche, insbesondere Kugeloberfläche durch die Kamera erzielen.

Es ist bekannt, daß ein Bild einer retroreflektierenden Kugel nicht eigentlich ein Bild eines Gegenstandes im üblichen Sinne ist, sondern eine Intensitätsverteilung der retroreflektierten Lichtmengen über die Kugeloberfläche aus Sicht der Kamera darstellt. Dementsprechend ist bei der erfindungsgemäßen Ausführungsform unter seitlich-schiefer Betrachtung bei den Korrekturmaßnahmen z.B. an der Außenwand des asphärischen Abschnitts der geringere Reflexionsverlust infolge eines weniger schrägen Lichtdurchgangs durch diesen Abschnitt gegenüber dem steigend schrägeren Lichtdurchgang durch den sphärischen Haubenabschnitt der transparenten Schutzhaube zu berücksichtigen.

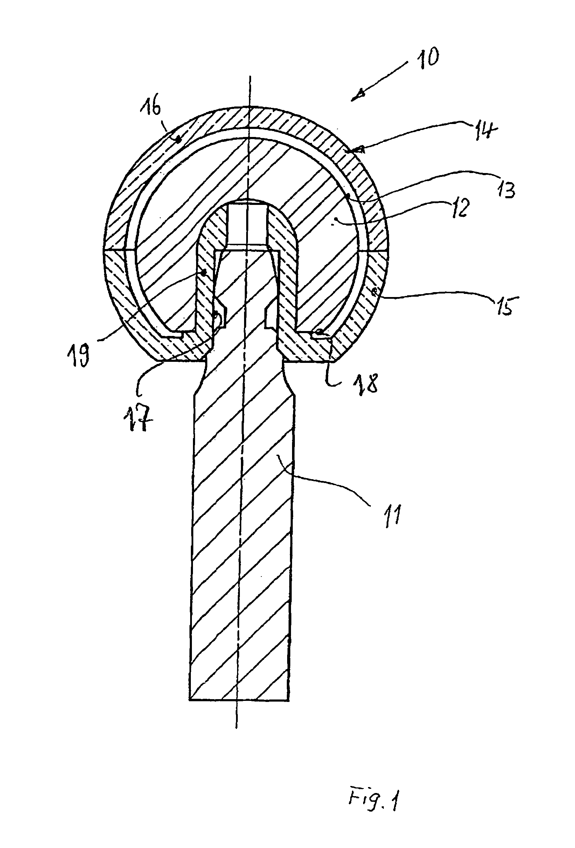

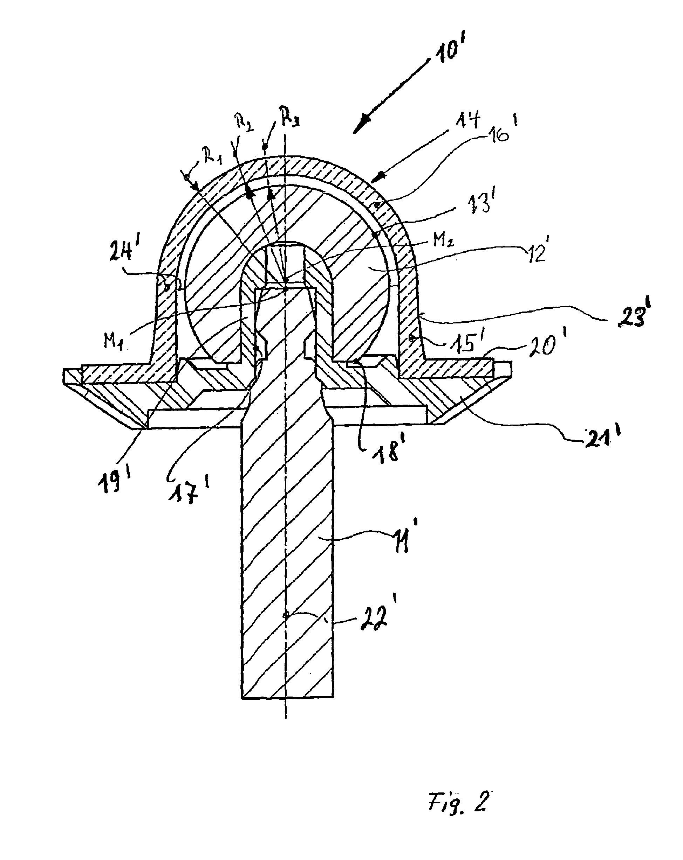

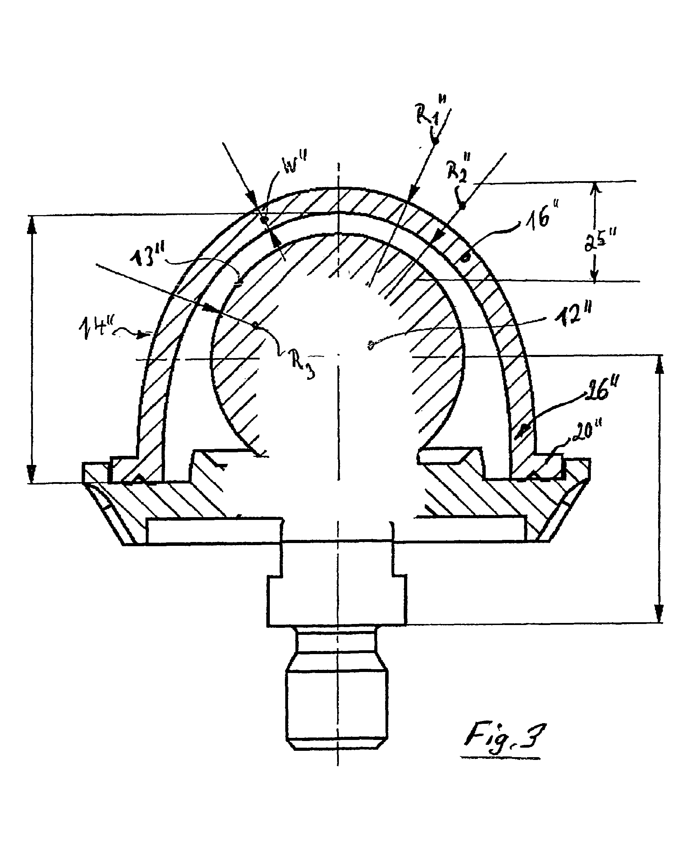

Nachstehend wird ein Ausführungsbeispiel eines erfindungsgemäß ausgebildeten Markers anhand der beigefügten Zeichnung näher erläutert, wobei die hier beanspruchte Erfindung im wesentlichen nur durch die Ausführungsform gemäß Fig. 3 verwirklicht ist, und die Ausführungsformen gemäß den Fig. 1 und 2 nur zur ergänzenden Erläuterung der Erfindung dienen. Dementsprechend zeigen:
- Fig. 1: eine erste Ausführungsform eines Markers im Längsschnitt;
- Fig. 2: eine zweite Ausführungsform eines Markers im Längsschnitt; und
- Fig. 3: eine dritte Ausführungsform eines Markers im Längsschnitt, der die Merkmale der beanspruchten Erfindung umfaßt.

In Fig. 1 ist ein Marker 10 für ein Navigationssystem zur Bestimmung der räumlichen Position z.B. eines chirurgischen Instruments im Längsschnitt und in vergrößertem Maßstab dargestellt. Dieser Marker 10 umfaßt eine Kugel 12 mit einer retroreflektierenden Oberfläche 13, die in an sich bekannter Weise feinste Glaskügelchen mit einem Durchmesser von 20 µ bis maximal etwa 200 µ umfaßt. Die retroreflektierende Oberfläche 13 ist durch eine formstabile, lichtdurchlässige bzw. transparente Schale 14, vorzugsweise aus transparentem Kunststoff, geschützt, und zwar derart, daß die reflektierte Abbildung hinsichtlich Kontur und Schwerpunktlage im wesentlichen unbeeinflußt bleibt. Wie oben ausgeführt, ist die Abbildung der retroreflektierenden Kugeloberfläche durch eine dem System zugeordnete Kamera idealerweise kreisrund. Dadurch, daß die Schale 14 die retroreflektierende Kugeloberfläche 13 in geringem Abstand konzentrisch umschließt und auch die Wandstärke der Schale 14 im Bereich der retroreflektierenden Oberfläche konstant ist, ergeben sich durch die Schale 14 keine Verzerrungen bei der Abbildung der retroreflektierenden Kugeloberfläche 13 durch eine dem System zugeordnete Kamera. Insbesondere läßt sich dadurch eine Abbildung der retroreflektierenden Kugeloberfläche sicherstellen, die idealerweise kreisrund ist.

Die transparente Schale 14, deren Wandstärke zwischen 0,7 mm bis 1,2 mm, insbesondere etwa 1,0 mm beträgt, ist von der retroreflektierenden Oberfläche 13 - wie bereits erwähnt - geringfügig beabstandet, wobei der Abstand zwischen etwa 0,1 mm bis 0,5 mm, insbesondere etwa 0,3 mm bis 0,4 mm beträgt. Der Spalt zwischen der retroreflektierenden Oberfläche 13 und der Schale 14 ist mit Luft oder Stickstoff gefüllt, d.h. mit einem Gas, dessen Brechungsindex etwa "1" beträgt. Das Gas ist maximal entfeuchtet, so daß die Gefahr irgend eines Feuchtigkeitsniederschlages oder einer Korrosion ausgeschlossen ist. Durch die Schale 14 ist auch die Kugel 12 der retroreflektierenden Oberfläche 13 gegenüber der äußeren Umgebung luftdicht gekapselt, so daß auch keine Feuchtigkeit in den Spalt zwischen der Kugel 12 und der Schale 14 eindringen kann.

Die transparente Schale 14 besteht bei der dargestellten Ausführungsform gemäß Fig. 1 aus zwei miteinander verkleb- oder verschweißbaren, insbesondere ultraschallverschweißbaren, und die retroreflektierende Oberfläche 13 bzw. die Kugel 12 konzentrisch umschließenden Schalenhälften 15, 16. Die untere Schalenhälfte 15 weist eine sacklochartige Ausnehmung 17 auf, in die ein einem nicht näher dargestellten Navigationssystem zugeordneter Trägerstift bzw. Steckdorn 11 einschiebbar ist.

Auf die das Sackloch 17 begrenzende Wandung der unteren Schalenhälfte 15 ist die Kugel 12 mit der retroreflektierenden Oberfläche 13 aufsteckbar. Dabei handelt es sich bei der Kugel 12 nicht um eine Vollkugel, sondern um eine an einer Seite abgeflachte Kugel. Mit der Abflachung 18 voraus ist die Kugel 12 auf die Sacklochwandung 19 aufgesteckt. Dabei ist die Passung zwischen Sacklochwandung 19 und Kugel 12 derart, daß ein luftdichter Abschluß zwischen diesen beiden Teilen sichergestellt ist. Auf diese Weise kann auch über das Sackloch 17 keine Feuchtigkeit in den Spalt zwischen Kugel 12 bzw. deren retroreflektierender Oberfläche 13 und Schale 14 von außen eindringen.

Die Ausführungsform gemäß Fig. 2 unterscheidet sich von derjenigen gemäß Fig. 1 dadurch, daß die die retroreflektierende Oberfläche 13' der Markerkugel 12' schützende Schale 14 als über die Kugel 12' gestülpte Haube mit einem sphärischen Haubenabschnitt 16' und einem sich daran anschließenden Zylinderabschnitt 15' ausgebildet ist. Die Schale 14 gemäß Fig. 2 ist also einstückig ausgebildet.

Am freien unteren Ende des Zylinderabschnitts 15 ist ein Flansch 20' angeformt, über den die Befestigung an einem Tragteller 21' erfolgt. Sofern der Tragteller 21' ebenso wie die Haube 14 aus Kunststoff besteht, erfolgt die Verbindung vorzugsweise durch Kleben oder Ultraschall-Schweißen. In dem Tragteller 21' ist zentrisch ein Sackloch 17' zur Aufnahme des Steckdorns 11' ausgebildet. Auf die das Sackloch 17' begrenzende Wandung 19' ist in der anhand der Fig. 1 beschriebenen Weise die Markerkugel 12' gesteckt, und zwar ebenfalls wieder mit der Abflachung 18' voraus. Die Verbindung zwischen der Haube 14 und dem Tragteller 21' ist luftdicht, so daß auch bei dieser Ausführungsform die Markerkugel 12 mit der retroreflektierenden Oberfläche 13' gegenüber der äußeren Umgebung abgekapselt ist. Auch hier kann keine Feuchtigkeit von außen in den Spalt zwischen der retroreflektierenden Oberfläche 13' und der Haube 14 eindringen.

Der Zylinderabschnitt 15' umfaßt vorzugsweise optische Korrekturbereiche zur Sicherstellung einer kreisrunden Abbildung der retroreflektierenden Oberfläche 13' auch nahe des maximalen Sichtwinkels relativ zur Mittenachse 22' der dargestellten Anordnung. Der maximale Sichtwinkel beträgt etwa 70° bis 75°. Auch in diesem Bereich soll noch gewährleistet sein, daß die Abbildung der retroreflektierenden Kugeloberfläche durch die zugeordnete Kamera des Systems kreisrund ist.

Konkret erstreckt sich zu diesem Zweck die äußere Umfangsfläche 23' des Zylinderabschnitts 15' in axialer Richtung ausgehend vom oder unterhalb des Übergangs 24' zwischen Zylinderabschnitt 15' und sphärischem Haubenabschnitt 16' konisch nach außen. Der Winkel zwischen der Mittenachse 22' und der sich konisch nach außen erstreckenden Umfangsfläche beträgt etwa 5° bis 12°, insbesondere etwa 8° bis 10°. Letztlich hängt dieser Winkel von dem maximalen Sichtwinkelbereich, dem Material und den Abmessungen von Kugel und Schutzhaube ab.

Bei der letztgenannten Ausführungsform ist auch noch von Interesse, daß der Mittelpunkt M₂ des Innenradius R₂ des sphärischen Haubenabschnitts 16' relativ zum Mittelpunkt M₁ des Außenradius R₁ desselben nach innen hin, d.h. in den sphärischen Haubenabschnitt 16' hinein verschoben ist, so wie dies in Fig. 2 dargestellt ist. Der Mittelpunkt M₂ ist auch der Mittelpunkt des Radius R₃ der retroreflektierenden Oberfläche 13'.

Der Abstand zwischen den sich auf der Mittenachse 22' befindlichen Mittelpunkte M₁ und M₂ beträgt etwa 0,08 mm bis etwa 0,12 mm, insbesondere etwa 0,1 mm, sofern die Radien R₁, R₂ und R₃ etwa wie folgt bemessen sind:
R₁ = 6,4 mm bis 7,0 mm
R₂ = 5,4 mm bis 6,0 mm
R₃ = 5,2 mm bis 5,7 mm.

Der sphärische Haubenabschnitt 16' der Ausführungsform gemäß Fig. 2 kann zur Sicherstellung einer kreisrunden Abbildung der retroreflektierenden Oberfläche 13' vorzugsweise auch asphärisch ausgebildet sein. Dies ist eine Frage der Feinabstimmung derart, daß die Forderung einer kreisrunden Abbildung der retroreflektierenden Kugeloberfläche durch eine dem System zugeordnete Kamera erfüllt wird.

Zu dem Material der Schale 14 sei noch erwähnt, daß der Brechungskoeffizient desselben etwa 1,5 betragen sollte.

Zu dem vorerwähnten Versatz zwischen den Mittelpunkten M1 und M2 sei auch noch erwähnt, daß dadurch eine vorbestimmte Brechungskorrektur erzielt wird, die durch die konkrete Ausführungsform gemäß Fig. 2 bedingt ist, d.h. durch die Kombination von halbkugelförmigem Haubenabschnitt 16' und Zylinderabschnitt 15'.

Die Ausführungsform gemäß Fig. 3 zeichnet sich dadurch aus, daß die vorgenannte Brechungskorrektur durch eine asphärische Haube 16" erhalten wird, die durchgehend gleiche Wandstärke W" aufweist. Im oberen Drittel 25" ist die Haube 16" sphärisch. Anschließend erweitert sich die Haube in Richtung zum Flansch 20" hin asphärisch kontinuierlich nach außen unter entsprechender Zunahme des äußeren und inneren Haubenradius R_{1''} und R_{2''}. Nahe des Flansches 20" nähert sich die Haube 16" asymptotisch einer Vertikalen (vertikal verlaufende Asphäre 26"). Die die retroreflektierende Oberfläche definierende Kugel ist mit der Bezugsziffer 12" gekennzeichnet.

### Bezugszeichen:

- 10, 10': Marker
- 11, 11': Steckdorn
- 12, 12', 12": Kugel
- 13, 13', 13": retroreflektierende Oberfläche
- 14, 14": Schale
- 15: untere Schalenhälfte
- 15': Zylinderabschnitt
- 16: obere Schalenhälfte
- 16', 16": sphärischer Haubenabschnitt
- 17, 17': Sackloch
- 18, 18': Abflachung
- 19, 19': Sacklochwandung
- 20', 20": Flansch
- 21': Tragteller
- 22': Mittenachse
- 23': äußere Umfangsfläche des Zylinderabschnitts 15'
- 24': Übergang zwischen Zylinderabschnitt 15' und Haubenabschnitt 16'
- 25": sphärischer Haubenabschnitt
- 26": vertikal verlaufende Asphäre

## Patentansprüche

1. Marker (10, 10') für ein Navigationssystem zur Bestimmung der räumlichen Position z.B. eines chirurgischen Instruments, mit einer sphärischen, retroreflektierenden Oberfläche (13"), insbesondere in Form einer retroreflektierenden Kugel (12"),
wobei die retroreflektierende Oberfläche (13") durch eine formstabile, lichtdurchlässige bzw. transparente Schale (14") geschützt ist, derart, daß die reflektierte Abbildung hinsichtlich Kontur und Schwerpunktlage im wesentlichen unbeeinflußt bleibt,
**dadurch gekennzeichnet, daß**
die transparente Schale (14") eine über die retroreflektierende Oberfläche (13") bzw. Kugel (12") gestülpte durchgehend gleiche Wandstärke (W") aufweisende Haube (16"), mit einem im oberen Drittel sphärischen Haubenabschnitt (25") ist, der sich anschließend in Richtung zu einem Flansch (20") hin asphärisch kontinuierlich nach außen unter entsprechender Zunahme des äußeren und inneren Haubenradius (R₁", R₂") erweitert.

2. Marker nach Anspruch 1,
**dadurch gekennzeichnet, daß**
die transparente Schale (14") von der retroreflektierenden Oberfläche (13") im Bereich zwischen 0,1 mm bis 0,5 mm, insbesondere im Bereich zwischen etwa 0,3 mm bis 0,4 mm beabstandet ist.

3. Marker nach Anspruch 1 oder 2,
**dadurch gekennzeichnet, daß**
die eine, insbesondere untere Schalenhälfte (15) eine sacklochartige Aufnahme (17) für einen Trägerstift bzw. Steckdorn (11) umfaßt, auf deren Begrenzungswand (19) die retroreflektierende Kugel (12) aufgesteckt ist.

4. Marker nach einem der Ansprüche 1 bis 3,
**dadurch gekennzeichnet, daß**
über den Flansch (20") die Befestigung an einem Tragteller (21') erfolgt, an den ein Trägerstift bzw. Steckdorn (11') anschließbar ist.

5. Marker nach einem der Ansprüche 1 bis 4,
**dadurch gekennzeichnet, daß**
der Spalt zwischen der retroreflektierenden Oberfläche (13"), insbesondere zugeordneten Kugel (12") und der transparenten Schale (14") mit Luft, Stickstoff od. dgl. inertem Gas gefüllt ist, also mit einem Gas mit einer optischen Brechzahl von nahe "1".

6. Marker nach einem der Ansprüche 1 bis 5,
**dadurch gekennzeichnet, daß**
die retroreflektierende Oberfläche (13"), insbesondere die retroreflektierende Kugel (12") durch die transparente Schale (14") luftdicht gegenüber der Umgebung abgekapselt ist.

## Claims

1. Marker (10, 10') for a navigation system for determining the position in space of for example a surgical instrument, the marker comprising a spherical, retroreflecting surface (13"), in particular in the form of a retroreflecting sphere (12"),
the retroreflecting surface (13") being protected by a dimensionally stable, translucent or transparent shell (14") such that the reflected image remains substantially unaffected in respect of shape and location of the centre of mass,
**characterised in that**
the transparent shell (14") is a cap (16") which is pulled over the retroreflecting surface (13") or sphere (12"), has a continuously uniform wall thickness (W"), and comprises a spherical cap portion (25") in the upper third, which cap portion then expands outwards towards a flange (20") in a continuous and aspheric manner, increasing the outer and inner cap radii (R₁"; R₂") accordingly.

2. Marker according to claim 1, **characterised in that** the transparent shell (14") is spaced apart from the retroreflecting surface (13") by a range of between 0.1 mm to 0.5 mm, in particular by a range of between approximately 0.3 mm to 0.4 mm.

3. Marker according to either claim 1 or claim 2, **characterised in that** one, in particular lower, shell half (15) comprises a blind-hole-like recess (17) for a centre pin support or insertion mandrel (11), on the delimiting wall (19) of which recess the retroreflecting sphere (12) is placed.

4. Marker according to any of claims 1 to 3, **characterised in that** fastening to a support plate (21') takes place via the flange (20"), to which support plate a centre pin support or insertion mandrel (11') can be connected.

5. Marker according to any of claims 1 to 4, **characterised in that** the gap between the retroreflecting surface (13"), in particular the associated sphere (12"), and the transparent shell (14") is filed with air, nitrogen or a similar inert gas, that is to say with a gas having an optical refractive index of approximately 1.

6. Marker according to any of claims 1 to 5, **characterised in that** the retroreflecting surface (13"), in particular the retroreflecting sphere (12"), is encapsulated by the transparent shell (14") in an air-tight manner relative to the environment.

## Revendications

1. Marqueur (10, 10') pour un système de navigation pour la détermination de la position spatiale, par exemple d'un instrument chirurgical, avec une surface sphérique rétro-réfléchissante (13"), plus particulièrement sous la forme d'une sphère rétro-réfléchissante (12"),
la surface rétro-réfléchissante (13") étant protégée par une coque rigide translucide ou transparente (14") de façon à ce que l'image réfléchie reste essentiellement inchangée en ce qui concerne le contour et la position du centre de gravité,
**caractérisé en ce que**
la coque transparente (14") est un capot (16") présentant une épaisseur de paroi (W") uniforme continue repliée sur la surface (13") ou la sphère (12") rétroréfléchissante, avec une portion de capot (25") sphérique dans le tiers supérieur, qui s'élargit en suite en direction d'une bride (20") de manière asphérique et continue vers l'extérieur avec une augmentation correspondante du rayon extérieur et intérieur (R₁" ; R₂") du capot.

2. Marqueur selon la revendication 1,
**caractérisé en ce que**
la coque transparente (14") est distante de la surface rétro-réfléchissante (13") de l'ordre de 0,1 mm à 0,5 mm, plus particulièrement de l'ordre d'environ 0,3 mm à 0,4 mm.

3. Marqueur selon la revendication 1 ou 2,
**caractérisé en ce que**
la moitié de coque (15), plus particulièrement inférieure, comprend un logement (17) en forme de trou borgne pour une tige de support ou une broche (11) sur la paroi de limitation (19) duquel la sphère rétro-réfléchissante (12) est insérée.

4. Marqueur selon l'une des revendications 1 à 3,
**caractérisé en ce que**
la bride (20') permet la fixation à un plateau de support (21'), auquel une tige de support ou une broche (11') peut être raccordée.

5. Marqueur selon l'une des revendications 1 à 4,
**caractérisé en ce que**
l'interstice entre la surface rétro-réfléchissante (13"), plus particulièrement la sphère (12") correspondante, et la coque transparente (14"), est rempli d'air, d'azote ou d'un autre gaz inerte, donc avec un gaz avec un indice de réfraction optique proche de 1.

6. Marqueur selon l'une des revendications 1 à 5,
**caractérisé en ce que** la surface rétro-réfléchissante (13"), plus particulièrement la sphère rétro-réfléchissante (12") est encapsulée de manière étanche à l'air par rapport à l'environnement par la coque transparente (14").
